# EUROPÄISCHE PATENTSCHRIFT

(11) **EP 1 720 806 B1**
(45) Veröffentlichungstag und Bekanntmachung des Hinweises auf die Patenterteilung: **08.07.2015**
(21) Anmeldenummer: 04804349.1
(22) Anmeldetag: 28.12.2004
(51) Int. Cl.: A61K 6/00, A61K 6/02, C03C 3/06, C03C 3/095, C03C 4/00, A61K 6/027, C03C 4/08

(54) **RÖNTGENOPAKES GLAS, VERFAHREN ZU SEINER HERSTELLUNG UND SEINER VERWENDUNG**
X-RAY OPAQUE GLASS, METHOD FOR THE PRODUCTION AND USE THEREOF
VERRE OPAQUE AUX RAYONS X, SON PROCEDE DE FABRICATION ET SON UTILISATION

(30) Priorität: 04.03.2004 DE 102004011218
(43) Veröffentlichungstag der Anmeldung: 15.11.2006
(73) Patentinhaber: Schott AG, 55122 Mainz (DE)
(72) Erfinder: PEUCHERT, Ulrich, 55294 Bodenheim (DE); KOLBERG, Uwe, 55130 Mainz (DE); BESINGER, Jörn, 84032 Landshut (DE)
(86) Internationale Anmeldenummer: PCT/EP2004/014760
(87) Internationale Veröffentlichungsnummer: WO 2005/085147

(56) Entgegenhaltungen:
- WO-A-98/58884
- WO-A-03/031355
- DE-A1- 3 206 227
- DE-A1- 19 849 388

## Beschreibung

Die Erfindung betrifft ein röntgenopakes Dentalglas, ein Verfahren zu seiner Herstellung und seine Verwendung.

Im Dentalbereich werden für die Zahnrestauration zunehmend Kunststoff-Dentalmassen eingesetzt. Diese Kunststoff-Dentalmassen bestehen üblicherweise aus einer Matrix aus organischen Harzen und verschiedenen anorganischen Füllstoffen. Die organischen Füllstoffe bestehen überwiegend aus Pulvern von Gläsern, (Glas-) Keramiken, Quarz oder anderen kristallinen Stoffen (z.B. YbF₃), Sol-Gel-Materiallen oder Aerosilen.

Durch die Verwendung von Kunststoff-Dentalmassen sollen mögliche schädliche Nebenwirkungen von Amalgam vermieden sowie ein verbesserter ästhetischer Eindruck erzielt werden. Abhängig von der Auswahl der Kunststoff-Dentalmassen können sie für unterschiedliche Zahnrestaurationsmaßnahmen verwendet werden, beispielsweise für Zahnfüllungen und auch für Befestigungen wie Kronen, Brücken und Inlays.

Das Füllmaterial als solches soll beim Aushärten den durch die Polymerisation der Harz-Matrix bedingten Schrumpf minimieren. Liegt beispielsweise eine starke Adhäsion zwischen Zahnwand und Füllung vor, kann ein zu großer Polymerisationsschrumpf zu einem Bruch der Zahnwand führen. Ist die Adhäsion hierfür nicht ausreichend, kann ein zu großer Polymerisationsschrumpf die Bildung von Randspalten zwischen Zahnwand und Füllung bewirken, welche Sekundärkaries fördern können. Darüber hinaus werden an die Füllstoffe bestimmte physikalische und chemische Anforderungen gestellt:
Das Füllmaterial muß zu möglichst feinen Pulvern zu verarbeiten sein. Je feiner das Pulver ist, desto homogener ist das Erscheinungsbild der Füllung. Gleichzeitig verbessert sich die Polierbarkeit der Füllung, was über die Verminderung der Angriffsfläche zu einer verbesserten Abrasionsfestigkeit und dadurch zu einer längeren Haltbarkeit der Füllung führt. Damit die Pulver gut zu verarbeiten sind, ist es darüber hinaus wünschenswert, wenn die Pulver nicht agglomerieren. Dieser unerwünschte Effekt tritt insbesondere bei Follmaterialien auf, die mit Hilfe von Sol-Gel-Verfahren hergestellt wurden.

Weiterhin ist es vorteilhaft, wenn der Füllstoff mit einem funktionalisierten Silan beschichtet wird, da dadurch die Formulierbarkeit der Dentalmasse erleichtert wird und die mechanischen Eigenschaften verbessert werden.

Darüber hinaus soll die Kunststoff-Dentalmasse und damit auch der Füllstoff hinsichtlich ihrer Brechzahl und Farbe möglichst gut an das natürliche Zahnmaterial angepaßt sein, damit sie möglichst wenig von dem umliegenden gesunden Zahnmaterial unterschieden werden kann. Für dieses ästhetische Kriterium spielt ebenfalls eine möglichst kleine Korngröße des pulverisierten Füllstoffs eine Rolle.

Weiterhin wichtig ist, dass die thermische Ausdehnung der Kunststoff-Dentalmasse im Verwendungsbereich, d.h. üblicherweise zwischen -30°C und +70°C, derjenigen des natürlichen Zahnmaterials angepaßt ist, um eine ausreichende Temperatur-Wechselbeständigkeit der Zahnrestaurationsmaßnahme zu gewährleisten. Auch durch eine zu hohe thermische Wechselbelastung können Spalte zwischen den Kunststoff-Dentalmassen und dem umliegenden Zahnmaterial entstehen, die wiederum bevorzugte Angriffspunkte für Sekundärkaries darstellen können. In der Regel werden Füllstoffe mit einem möglichst geringen thermischen Ausdehnungskoeffizienten verwendet, um die große thermische Ausdehnung der Harz-Matrix zu kompensieren.

Eine gute chemische Beständigkeit der Füllstoffe gegenüber Säuren, Laugen und Wasser sowie eine gute mechanische Stabilität bei Belastungen wie z.B. der Kaubewegung kann darüber hinaus zu einer langen Lebensdauer der Zahnrestaurationsmaßnahmen beitragen. ,

Für die Behandlung von Patienten ist es ferner unbedingt erforderlich, daß Zahnrestaurationsmaßnahmen im Röntgenbild sichtbar sind. Da die Harz-Matrix selbst im Röntgenbild in der Regel unsichtbar ist, müssen die Füllstoffe für die notwendige Röntgenabsorption sorgen. Ein solcher Füllstoff, der Röntgenstrahlung ausreichend absorbiert, wird röntgenopak genannt. Für die Röntgenopazität sind in der Regel Bestandteile des Füllstoffes, beispielsweise bestimmte Komponenten eines Glases, oder Zusatzstoffe verantwortlich, sogenannte Röntgenopaker. Ein gebräuchlicher Röntgenopaker ist YbF₃, welches in kristalliner, gemahlener Form dem Füllstoff zugesetzt werden kann.

Weil die Kunststoff-Dentalmasse in der Anwendung üblicherweise aus Kartuschen in Kavitäten eingefüllt und dort modelliert wird, soll sie häufig im nicht ausgehärteten Zustand thixotrop sein. Das heißt, dass ihre Viskosität beim Ausüben von Druck abnimmt, während sie ohne Druckeinwirkung formstabil ist.

Bei den Kunstsoff-Dentalmassen sind weiterhin Dentalzemente und Komposite zu unterscheiden. Bei Dentalzementen, auch Glasionomerzemente genannt, führt die chemische Reaktion der Füllstoffe mit der Harz-Matrix zum Aushärten der Dentalmasse, weshalb durch die Reaktivität der Füllstoffe die Aushärtungseigenschaften der Dentalmasse und damit deren Bearbeitbarkeit beeinflußt wird. Es handelt sich hierbei oftmals um einen Abbindevorgang, dem ein radikalisches oberflächiges Aushärten , beispielsweise unter der Einwirkung von UV-Licht, vorausgeht. Komposite, auch Füllungskomposite genannt, enthalten dahingegen weitergehende chemisch weitestgehend inerte Füllstoffe, da ihre Aushärteverhalten durch Bestandteile der Harz-Matrix selbst bestimmt werden und eine chemische Reaktion der Füllstoffe hierfür oftmals störend ist.

Weil Gläser aufgrund ihrer unterschiedlichen Zusammensetzungen eine Werkstoffklasse mit vielfältigen Eigenschaften repräsentieren, werden sie häufig als Füllstoffe für Dentalmassen eingesetzt. Solche Gläser werden allgemein Dentalgläser genannt. Reaktive Dentalgläser zur Verwendung in Dentalzementen sind beispielsweise aus der DE 100 63 939 A1 bekannt.

DE 32 06 227 A1 beschreibt ein ophthalmisches Glas, das nennenswerte Anteile von B₂O₃ enthält. Die WO 98/58884 A offenbart ein Glas für optische Verstärker, welches seltene Erden und nennenswerte Anteile an Al₂O₃ beinhaltet. Diese Gläser sind für Dentalanwendungen ungeeignet.

Chemisch inerte Dentalgläser zur Verwendung als Füllstoff in Kompositen sind Gegenstand der DE 198 49 388 A1. Die dort vorgeschlagenen Gläser enthalten zwingend nennenswerte Anteile an Al₂O₃, ZnO, F und Na₂O, wodurch deren chemische Beständigkeit negativ beeinflusst wird. Ferner kann der F-, ZnO- und Na₂O-Gehalt zu Reaktionen mit der Harz-Matrix führen, was wiederum Auswirkungen auf deren Polymerisationsverhalten haben kann. Als weiterer Bestandteil ist in den der DE 198 49 388 A1 zugrundegelegten Gläsern zwingend ZrO₂ vorhanden, um Röntgenopazität zu bewirken. Derartige Füllstoffe sind zu reaktiv insbesondere für modernste Dentalmassen auf Epoxibasis, bei denen ein zu schnelles, unkontrolliertes Aushärten erfolgen kann.

Aus der DE 101 00 680 A1 sind Dentalgläser bekannt, die mindestens zwei Komponenten enthalten. Diese binären Glassysteme bestehen aus einem hohen Anteil von SiO₂ und HfO₂, TaO₆, ZrO₂ oder La₂O₃, welche die Röntgenopazität bewirken. Allerdings wurden nur die binären Gläser aus SiO₂ und HfO₂ sowie SiO₂ und Ta₂O₆ durch einen Schmelzprozeß erzeugt, während die Gläser aus SiO₂ und ZrO₂ bzw. La₂O₃ mit Hilfe eines Sol-Gel-Prozesses erhalten wurden. Der Sol-Gel-Prozeß ist beispielsweise von Nogami in Journal of Non-Crystalline Solids, 96 (1985) 415-423 beschrieben. Er hat wirtschaftlich den Nachteil, dass er zu teuer für die Herstellung größerer Mengen von Dentalgläsern ist. Darüber hinaus führen durch den Sol-Gel-Prozeß hergestellte Gläser meist viel Wasser mit sich, was ihre Weiterverarbeitung zu Pulvern erschwert. Insbesondere neigen Sol-Gel-Glaspulver oftmals zu Agglomeration. Ferner werden in der DE 101 00 680 A1 tenäre Glassysteme vorgeschlagen, die neben SiO₂ als Hauptbestandteil ZrO₂ als röntgenopaken und La₂O₃, HfO₂, Y₂O₃, TiO₂ und Al₂O₃ als weitere Bestandteile enthalten. Auch das ternäre System aus SiO₂, La₂O₃ und B₂O₃ wird beschrieben.

Die genannten Gläser haben den Nachteil, dass sie hinsichtlich ihrer Röntgenopazität, ihrer Verarbeitbarkeit, ihres Brechungsindex und ihres Herstellungsverfahrens nicht optimal für alle Anwendungen als Dentalglas geeignet sind. Daher ist es Aufgabe der vorliegenden Erfindung, ein röhtgenopakes Glas bereitzustellen, welches als reaktionsträger Füllstoff für Komposite geeignet ist und insbesondere wegen seiner Reaktionsträgheit einen Beitrag zur Langzeitstabilität von auf Epoxydharz basierenden Füllstoffen der neuesten Generation leistet. Diese Gläser sollen ferner die kostengünstige Herstellung von Dentalmassen ermöglichen, die ohne teure, kristallisierte Röntgenopaker und ohne auf Acrylat basierende Füllungszemente auskommen. Eine weitere Aufgabe der Erfindung ist die Bereitstellung eines wirtschaftlichen Verfahrens zur Herstellung der vorgenannten Gläser.

Die Aufgaben werden durch die unabhängigen Ansprüche gelöst. Bevorzugte Aüsführungsformen ergeben sich aus den Unteransprüchen. Das erfindüngsgemäße Glas enthält SiO₂ mit einem Anteil von 60 - 98 Mol-%, sowie Yb₂O₃ mit einem Anteil von 0,1 - 40 Mol% und kein Al₂O₃ und/oder B₂O₃. Es hat sich gezeigt, daß durch den Gehalt an Yb₂O₃ eine überraschend gute Röntgenopazität erzielt wird und die Brechungsindices dieser Gläser sehr gut an die natürliche Zahnanmutung anzupassen sind. Das erfindungsgemäße Glas kann ferner ZrO₂ bis zu einem Anteil von 40 Mol-% enthalten.

Bevorzugt enthält das erfindungsgemäße Glas neben SiO₂ mit einem Anteil von 60 - 98 Mol-% und Yb₂O₃ von 0,1 - 40 Mol-% zusätzlich ZrO₂ mit einem Anteil von 0,1 - 40 Mol-%. Die Zugabe dieser dritten Komponente stabilisiert das Glas, so daß es eine geringere Neigung zu Kristallisation aufweist. Die Kristallisation eines Glases ist zu vermeiden, da sie u.a. die optischen Eigenschaften dermaßen beeinflussen würde, daß das Füllmaterial nur schwer oder überhaupt nicht an die natürliche Zusammensetzung anzupassen wäre.

In einer weiteren bevorzugten Ausführungsform weist das erfindungsgemäße Glas neben SiO₂ mit einem Anteil von 70 - 98 Mol-% und Yb₂O₃ von 0,5 - 15 Mol-% zusätzlich ZrO₂ mit einem Anteil von 0,5 - 15 Mol-% auf. Besonders bevorzugt werden Anteile von 70 - 98 Mol-% SiO₂, 1 - 15 Mol-% Yb₂O₃ und zusätzlich 1 - 15 Mol-% ZrO₂.

In einer ganz besonders bevorzugten Ausführungsform sind in dem erfindungsgemäßen Glas als zusätzliche Komponenten WO₃, La₂O₃, Nb₂O₅, HfO₂, Ta₂O₅, Gd₂O₃, Lu₂O₃, Sc₂O₃, Y₂O₃ mit jeweils bis zu 40 Mol-% und/oder F₂ bis zu 5 Mol-% enthalten. Der Anteil an F₂ kann durch die Zugabe von YbF₃ in das Gemenge erhalten werden, wobei dem Fachmann bekannt ist, daß Fluor in einem Glas nicht als gasförmige Komponente vorliegt. Durch die Kombination der Oxide dieser zusätzlichen schweren Elemente kann das Röntgenabsorptionsspektrum des erfindungsgemäßen Glases, das durch die Überlagerung der Röntgenabsorptionsspektren der einzelnen Komponenten entsteht, an das Emissionsspektrum verschiedener Röntgenquellen angepaßt werden. Auf diese Weise kann erreicht werden, daß geringere Strahlendosen für die Untersuchung von Patienten benötigt werden.

Um die Schmelzbarkeit der Gläser mit Hilfe von Hochfrequenzverfahren (s.u.) zu verbessern, kann das erfindungsgemäße Glas ferner die Alkalioxide Li₂O, Na₂O und/oder K₂O bis zu einem Anteil von jeweils 10 Mol-% enthalten, wobei deren Gesamtanteil höchstens 10 Mol-% betragen soll.

In einer bevorzugten Ausführungsform enthalten die erfindungsgemäßen Gläser die Erdalkalioxide MgO, CaO, SrO, BaO und/oder ZnO bis zu einem Anteil von jeweils 10 Mol-%, wobei deren Gesamtanteil wiederum höchstens 10 Mol% betragen soll. Die Erdalkalioxide fördern das Einschmelzverhalten sowie die Glasbildung und reduzieren die Kristallisationsneigung. Darüber hinaus können ZnO, SrO und BaO eine antibakterielle Wirkung entfalten und die Röntgenopazität verbessern.

Es hat sich gezeigt, daß trotz des Gehalts an Alkali- und/oder Erdalkalioxiden in der SiO₂-Yb₂O₃-Matrix des erfindungsgemäßen Glases dessen chemische Beständigkeit überraschend gut ist, woraufhin mit einer großen Reaktionsträgheit im Zusammenspiel mit der Harzmatrix und damit mit einer sehr guten Langlebigkeit der gesamten Dentalmasse zu rechnen ist. Es ist selbstverständlich auch möglich, die Farberscheinung des Glases durch die Zugabe von dazu gebräuchlichen Oxiden anzupassen.

Als zusätzliche Komponenten, welche bestimmte Glaseigenschaften wie Kristallisatiönsneigung, Schmelzbarkeit etc. verbessern können, wird vorgesehen, daß das erfindungsgemäße Glas TiO₂, GeO₂ und/oder P₂O₅ bis zu jeweils 10 Mol-% enthalten kann, wobei deren Gesamtgehalt bis zu 15 Mol-% betragen soll.

Durch die Beschränkung der Anzahl der Komponenten kann erreicht werden, daß die Wahrscheinlichkeit, daß Verunreinigungen das Glas kontaminieren, reduziert wird. Daher kann das erfindungsgemäße Glas für besondere Anwendungen bevorzugt nur höchstens fünf der zuvor genannten oxidischen Komponenten enthalten. Eine noch höhere Reinheit kann dadurch erzielt werden, daß die Auswahl der oxidischen Komponenten auf höchstens vier, besonders bevorzugt auf höchstens drei beschränkt wird.

Die Erfindung umfaßt darüber hinaus Glaspulver aus den vorgenannten Gläsern. Die Glaspulver werden durch bekannte Verfahren erzeugt, wie z.B. in der DE 41 00 604 C1 beschrieben. Das erfindungsgemäße Glaspulver weist bevorzugt eine mittlere Korngröße bis zu 20 µm auf. Eine mittlere Korngröße von 0,2 µm kann als Untergrenze erreicht werden, wobei natürlich auch kleinere Korngrößen von der Erfindung umfaßt werden. Das vorgenannte Glaspulver kann als Ausgangsmaterial für die Verwendung der erfindungsgemäßen Gläser als Füllstoffe dienen.

In einer bevorzugten Ausführungsform wird die Oberfläche des Glaspulvers mit den gebräuchlichen Methoden silanisiert. Durch die Silanisierung kann erreicht werden, daß die Bindung der anorganischen Füllstoffe an die Kunststoffmatrix der Dentalmasse verbessert wird.

Das erfindungsgemäße Glas weist einen sehr hohen Schmelzpunkt auf und kann nicht durch konventionelle Schmelzverfähren hergestellt werden. Daher ist das Bereitstellen eines für das Glas anzuwendenden Schmelzverfahrens ebenfalls Gegenstand der Erfindung. Schmelzverfahren zum Herstellen von Gläsern beinhalten im allgemeinen den Schritt der Gemengeaufbereitung, während dem die Rohstoffkomponenten den Anforderungen entsprechend verwischt, vorbehandelt, gereinigt etc. werden, den Schritt der Gemengeeinlage, wobei die aufbereiteten Rohstoffkomponenten in das Schmelzgefäß eingebracht werden, und dem eigentlichen Schmelzen. Andere Verfahren, z.B. Sol-Gel-Verfahren, sind für das Herstellen größerer Glasmengen nicht wirtschaftlich einzusetzen. Außerdem besteht insbesondere bei SiO₂ und ZrO₂ führenden und mit Sol-Gel-Verfahren hergestellten Gläsern die Gefahr der Agglomerierung der Pulver. Ein erfindungsgemäßes Verfahren ist das Hochtemperaturschmelzen. Die Temperatur der Glasschmelze beträgt dabei mindestens 1500°C, bevorzugt mindestens 1600°C.

Die beim Hochtemperaturschmelzen auftretenden Temperaturen stellen besondere Anforderungen an die für das Schmelzgefäß verwendeten Materialien. In einer bevorzugten Ausführungsform besteht das Schmelzgefäß daher zumindest teilweise aus massivem Iridium und/oder einer Legierung mit einem hohen Iridiumgehalt. Iridiumgehalte von mindestens 95% haben sich in solchen Legierungen als geeignet erwiesen. Aufgrund der Oxidationsempfindlichkeit des Iridiums ist eine solche Bauteile enthaltende Vorrichtung zumindest teilweise mit einem geeigneten Schutzgas zu spülen. Die Beheizung des Schmelzgefäßes kann beim Hochtemperaturschmelzen mit konventionellen Methoden erfolgen, beispielsweise mittels induktiver Beheizung mit einer Frequenz von etwa 8 kHz bis etwa 12 kHz. Ein solches Verfahren und eine Vorrichtung zum Durchführen des Verfahrens werden für andere Glassysteme in der DE 103 48 466 (noch nicht veröffentlicht), ausführlich beschrieben.

In einem anderen bevorzugten Verfahren werden die hohen Temperaturen während des Schmelzvorgangs durch das Einkoppeln hochfrequenter elektromagnetischer Wechselfelder in ein bereits zumindest teilweise verflüssigtes Glasgemenge erzeugt. Der Frequenzbereich kann üblicherweise zwischen 50 kHz und 2 MHz liegen. Dieses Verfahren wird Hochfrequenzschmelzen genannt. Solche Verfahren und entsprechende Vorrichtungen sind beispielsweise in der WO 01/14264 A1, WO 01/14265 A1 und WO 03/031355 A1 ausführlich beschrieben. Da der dem Hochfrequenzschmelzen zugrundeliegende Mechanismus allerdings erst ab einer bestimmten Temperatur wirksam wird, erfolgt vor dem Einschalten des Wechselfeldes üblicherweise eine Aufheizung mit konventionellen Heizverfahren, beispielsweise mit Brennern.

Durch Hochfrequenzschmelzen können prinzipiell beliebig hohe Temperaturen erreicht werden. Bis zu 2500 °C sind bereits mit kommerziell erhältlichen Laboranlagen zu erreichen. Noch höhere Temperaturen sind durch geeignete Modifikationen realisierbar. Das Hochtemperaturschmelzen kann bevorzugt verwendet werden, da es sich gezeigt hat, daß die Sauerstoff-Ionenleiter Yb₂O₃ und ZrO₂ als Reinsubstanz hervorragend an das elektromagnetische Wechselfeld ankoppeln. Bei Zumischen anderer Substanzen wie SiO₂, die nichtleitend sind, sinkt die Leitfähigkeit mit zunehmender Konzentration soweit ab, bis die Schmelze auskoppelt. Dieser Zusammensetzungspunkt ist nicht genau definiert, er hängt von apparativen Größen und dem zeitlichen Verlauf des Temperaturprofils der Schmelze ab. Um auch noch hoch SiO₂-haltige Zusammensetzungen mit Hilfe von Hochfrequenzeinstrahlung schmelzen zu können, hat es sich bewährt, geringe Mengen von Alkalioxiden zuzusetzen, dabei bevorzugt Li₂O und Na₂O bis zu 10 Mol-%. Diese erhöhen bereits in diesen niedrigen Konzentrationen die Leitfähigkeit und damit die Hochfrequenzschmelzbarkeit stark. ,

Die Frequenz des elektromagnetischen Wechselfeldes beim Hochfrequenzschmelzen ist abhängig vom zu schmelzenden Volumen. Sie kann während des Hochfrequenzschmelzens bevorzugt von 50 kHz bis 2 MHz betragen. Alternativ können Verfahren wie Plasmaschmelzen, Laserschmelzen oder Spiegelofentechnologie angewendet werden.

Alternative Verfahren zum hier beschriebenen Hochtemperaturschmelzen um-, fassen weiterhin Flammenhydrolyse, bei der Gasförmige Precursoren verwendet werden, oder Plasmasprühen, bei dem üblicherweise Pulver als Precursoren dienen.

Das erfindungsgemäße Glas besteht in der Hauptsache aus hochschmelzenden Rohstoffen, die wie im Falle von SiO₂, Yb₂O₃ und ZrO₂ im Anlieferungszustand relativ grobkörnig und reaktionsträge sind. Diese können in der üblichen Weise, d.h. durch Vermischen der pulverförmigen, kommerziell erhältlichen Ausgangsoxide und Einlegen in das Schmelzgefäß, erschmolzen werden. Dabei kann es aufgrund der Reaktionsträgheit und hohen Schmelztemperaturen der Ausgangsoxide sowie der hohen Viskosität des entstehenden Glases aufgrund kinetischer Hemmung zu einem sehr langsamen Fortschreiten des Schmelzprozesses kommen. Mit anderen Worten: die Temperatur wäre zwar prinzipiell hoch genug, um eine gewünschte Glaszusammensetzung nahe des Eutektikums der Ausgangskomponenten zu schmelzen, nicht jedoch für die Ausgangsoxide selbst. Deswegen liegen in diesem Fall weitestgehend feste Substanzen vor, die nur über sehr langsame fest-fest-Reaktionsprozesse allmählich miteinander reagieren. Die Glasbildung schreitet in diesem Fall nur langsam voran, da Stofftransporte über relativ große Entfernungen stattfinden müssen. Um für die Glasproduktion wirtschaftlich angemessene Reaktionsraten zu erzielen, muß die Temperatur weit über das thermodynamisch notwendige Maß hinaus angehoben werden. Dies ist aus energiewirtschaftlichen und/oder apparativen Gründen jedoch oftmals nachteilig bzw. unmöglich. Weiterhin weist die Schmelze in dem Größenbereich, der den ehemaligen Korngrößenabmessungen entspricht, Kornzentrationsinhomogenitäten auf, die sich auch bei Rühren nur langsam abbauen. Es hat sich gezeigt, daß der Einschmelzvorgang wirtschaftlicher - das bedeutet mit weniger Energieaufwand und schneller - erfolgen kann, wenn zumindest eine der Rohstoffkomponenten des erfindungsgemäßen Glases in nanoskaliger oder wasserlöslicher Form vorliegt. Unter nanoskalig werden Pulver mit sehr kleinen Korngrößen, üblicherweise von 10 nm bis 200 nm verstanden. Die mindestens eine nanoskalige Rohstoffkomponente kann mit den anderen, ggfls. gemahlenen Rohstoffkomponenten als Gemenge in das Schmelzgefäß eingelegt werden.

Bevorzugt werden die Rohstoffkomponenten jedoch in der Gemengeaufbereitung, wie unten beschrieben, zu sogenannten Grünkörpern oder Grünkörperpulvern vorbehandelt. Durch die Aufbereitung der Rohstoffkomponenten zu einem Grünkörper werden die Festkörperreaktionen beim Einlegen in das Schmelzgefäß wesentlich beschleunigt, da die Diffusionswege um mehr als eine Größenordnung kleiner sind als wenn bei der Gemengeeinlage von keinem Grünkörper ausgegangen würde. Weiterhin haben nanoskalige Rohstoffkomponenten ein günstigeres Verhältnis von reaktiver Oberfläche zu wenig reaktivem Volumen. Die Reaktionen laufen wesentlich schneller ab, bzw. bei vergleichbarer Reaktionsgeschwindigkeit kann die Temperatur um etwa 500°C bis 700°C niedriger gewählt werden. Dadurch werden manche Glaszusammensetzungen überhaupt erst technisch schmelzbar. Des weiteren liegen die oben erwähnten Konzentrationsinhomogenitäten in diesem Fall nicht im µm-Bereich, sondern im Bereich einiger nm (beispielsweise etwa 10 nm), so daß auch die Rührprozesse zur Homogenisierung wesentlich schneller und effektiver ablaufen.

Die Herstellung der zuvor genannten Grünkörper kann auf vielfältige Weise erfolgen. Bevorzugt werden in einem kommerziell erhältlichen Dissolver, welcher im Prinzip ein Rührwerk mit hoher Umdrehungszahl, speziellen stark scherenden Rührwerkzeugen und Einrichtungen zur Temperaturkontrolle darstellt, in einer vorgegebenen Menge eines Lösungsmittels, beispielsweise Wasser, zunächst die löslichen Komponenten gelöst. Danach wird unter starkem Rühren die nanoskalige Komponente, beispielsweise SiO₂, in Form von Pulver oder einer vorgefertigten Suspension zugegeben. Die Zugabe der anderen bevorzugt nanoskaligen oder aber auch grobkristallinen Substanzen erfolgt im Anschluß. Die Temperatur wird dabei vorteilhaft möglichst niedrig gehalten. Sofern Reaktionswärme auftritt, kann diese durch eine Kühlvorrichtung abgeführt werden. Rührgeschwindigkeit und Rührzeit zur Erzielung einer möglichst homogenen Komponentenverteilung richten sich nach der Art der chemischen Zusammensetzung der Masse und den erhältlichen Korngrößen. Die dabei erhaltene fließfähige bis pastöse Suspension wird in Formen geeigneter Größe gegossen und/oder gestrichen.

Die in den Formen befindliche Masse kann nachfolgend getrocknet werden. Dafür sind übliche Trockenschränke oder Kammeröfen geeignet. Besonders bevorzugt erfolgt die Trocknung allerdings in Mikrowellentrockenanlagen, wobei bevorzugt ein Temperaturbereich von 100°C bis 250°C gewählt wird. Höhere Temperaturen sind prinzipiell möglich, können aber zu Verbackungen führen. Es wurde festgestellt, daß durch die Verwendung einer Mikrowellenanlage zur Trocknung in sehr kurzen Trocknungszeiten eine ausreichende Menge getrockneter Grünkörper erhalten wird. Ferner werden Entmischungen durch Mitschleppeffekte in dem im konventionellen Fall zur Oberfläche diffundierenden Lösungsmittel vermieden. Höchst überraschend wurde ebenfalls festgestellt, daß durch die Mikrowellentrocknung weniger Gaseinschlüsse und damit unerwünschte Blasen beim Einschmelzen des Grünkörpers und/oder des Grünkörperpulvers entstehen.

In einer bevorzugten Ausgestaltung des Verfahrens besteht die Form, in der die Suspension eingefüllt und der Grünkörper getrocknet wird, zumindest teilweise aus einem nicht benetzenden Material. Bevorzugt wird hierbei Teflon verwendet. Als Folge dieser Materialwahl wird die Trocknungstemperatur auf eine Obergrenze von etwas 250°C bis 300°C begrenzt. Teflon ist ferner weitestgehend chemisch inert gegenüber dem Trockengut. Dadurch können Verunreinigungen vermieden werden. Es hat sich gezeigt, daß der Grünkörper bei Verwendung von nicht benetzendem Material in der Form sehr gut aus diesen entfernt werden kann. Im Falle von Trockengefäßen aus Glas oder Metall kann es durch die erwünschte hohe Reaktivität des Grünköpers zu Angriffen auf die Form kommen, die zum einen zu Verunreinigungen führen können, zum andern zu einer festen Verbackung zwischen Grünkörper und Form, so daß sich der Grünköper nur noch schwer oder überhaupt nicht mehr herauslösen läßt.

Nach erfolgter Trocknung können die Grünkörper als Ganzes, als grobe Fragmente oder gemahlen als Gemenge in das Schmelzgefäß eingelegt werden.

Zur weiteren Steigerung der Homogenität des Gemenges kann der Grünkörper jedoch nochmals gemahlen, dispergiert und der gesamte zuvor beschriebene Prozeß zum Herstellen eines Grünköpers nochmals durchlaufen werden. Der nochmals aufbereitete Grünkörper wird Kompaktkörper genannt. Beim Mahlen können auch Scherben aus früheren Schmelzen zur Wiederverwertung zugesetzt werden.

Bei der Herstellung der Grün- und/oder Kompaktkörper ist es im allgemeinen vorteilhaft, Alkalihydroxide zuzusetzen und somit im basischen Medium zu arbeiten, d.h. in der Regel mit einer Alkalilauge. In diesem Fall kommt es durch Auflösungs- und Wiederanlagerungsprozesse des nanoskaligen SiO₂ und/oder der übrigen festen nanoskaligen Substanzen sowie der Fällung wasserlöslicher Verbindungen als Hydroxid zu einer Vermischung der Komponenten auf nahezu monomolekularer Ebene. Auf diese Weise verschwinden sozusagen die ursprünglichen Korngrenzen und es entsteht ein sehr einheitlicher, gut abbindender und leicht sinter- und schmelzbarer Grünkörper. Als Folge ist das Glas dann allerdings nicht mehr alkalifrei. Der Anwender hat im Einzelfall abzuwägen, ob der Nutzen durch die deutlich besseren Prozeßabläufe, die im allgemeinen geringe Verschlechterung der chemischen Beständigkeit kompensiert. Vorteilhaft ist die Verwendung von Alkalioxiden insbesondere dann, wenn der eigentliche Schmelzvorgang unter Hochfrequenzschmelzen abläuft, da die Alkalioxide wie zuvor beschrieben die Koppelfähigkeit der Schmelze verbessern können.

Statt Alkalioxiden kann auch eine wässrige Ammoniaklösung verwendet werden. Allerdings ist der pH-Wert bei weitem nicht so basisch wie bei den Alkalioxiden, die zuvor beschriebenen Reaktionen laufen dann weniger ausgeprägt ab. Weiterhin kann Stickstoff, beispielsweise als Nitrid, im Glas verbleiben. Es ist im Einzelfall je nach Anwendung abzuwägen, ob diese, wenn auch geringen Mengen, stören können, beispielsweise durch Verfärbungen.

Es ist ebenfalls möglich, die Grün- oder Kompaktkörper nicht direkt in das Schmelzgefäß einzulegen, sondern einer Sinterung zu unterziehen. Dies kann beispielsweise in Kammeröfen, Tunnelöfen oder Drehrohröfen bei Temperaturen zwischen 700°C und 1600°C geschehen. Dies hat den Vorteil, daß die Grün- oder Kompaktkörper zu Sinterkörpern verdichtet werden. Wird ein Sinterkörper zum Schmelzen eingelegt, kann dies den Vorteil haben, daß die Wärmeübertragung in der Schmelze und damit die Schmelzbarkeit verbessert wird. Darüber hinaus bewirkt die Sinterung eine Volumenkompaktierung und dadurch einen geringeren Platzbedarf bei der Lagerung sowie eine verbesserte chemische Stabilität des Lagerguts. In einem Sinterkörper sind ferner weniger eingeschlossene Gase enthalten und beim Einlegen entsteht weniger Staub als beim Einlegen von Pulvern. Enthält das Gemenge Carbonate und/oder Nitrate, ist die Sinterung vorteilhaft, weil diese Komponenten dazu neigen, bei Erhitzung Gase freizusetzen, Insbesondere Nitrate können jedoch das Schmelzgefäß angreifen. Werden die Grün- oder Kompaktkörper gesintert, erfolgt die Gasfreisetzung bereits während der Sinterung, weshalb der Anteil der gasfreisetzenden Komponenten im Gemenge reduziert wird und der Sinterkörper über längere Zeit eingelagert werden kann als ein Grün- oder Kompaktkörper. Darin können produktionstechnische Vorteile begründet sein. Ferner kann das i.d.R. teure Schmelzgefäß, in dem sehr hohe Temperaturen erreicht werden müssen, von vorgelagerten Reaktionen entlastet werden, wenn Sinterkörper eingelegt werden, da diese Vorreaktionen bereits während der Sinterung stattgefunden haben. Außerdem kann das Schmelzgefäß auf diese Weise vor den genannten schädigenden Substanzen, beispielsweise Nitraten, geschützt werden. Als Folge wird es so möglich, preiswertere Schmelzgefäße zu verwenden.

In einer besonders bevorzugten Ausgestaltung des Verfahrens erfolgt die Sinterung unmittelbar vor der Einlage in das Schmelzgefäß. In einer entsprechenden Schmelzanlage befindet sich die Einrichtung zum Sintern direkt in Linie vor der Einrichtung zum Schmelzen. Die Abwärme der Schmelzeinrichtung kann auf diese Weise während des Sintervorgangs zum Vorheizen der Grün- oder Kompaktkörper auf Reaktionstemperatur beitragen und der Wärmeinhalt des Sinterkörpers geht bei der Einlage in das Schmelzgefäß nicht verloren.

Das erfindungsgemäße Glas wird als Dentalglas eingesetzt. Bevorzugt findet es Anwendung als Füllstoff in Kompositen für die Zahnrestauration, besonders bevorzugt für auf Epoxydharz basierende Füllstoffe, die weitgehend chemisch inerte Füllstoffe erfordern. Ebenfalls im Sinne der Erfindung ist die Verwendung des erfindungsgemäßen Glases als Röntgenopaker in Dentalmassen. Das Glas ist geeignet, teure kristalline Röntgenopaker wie beispielsweise YbF₃ zu ersetzen.

Beispiele der erfindungsgemäßen Gläser sind in Tabelle 1 aufgelistet. Beispiel Nr. 38 ist ein aus der DE 101 00 680 A1 bekanntes Glas, das dort mit einem Sol-Gel-Verfahren und hier durch Hochtemperaturschmelzen erzeugt wurde. Beispiel Nr. 37 ist ebenfalls ein binäres Glas aus den Komponenten SiO₂ und Zr0₂, das durch Hochtemperaturschmelzen erzeugt wurde. Die Beispiele Nr. 37 und 38 dienen als Vergleichsbeispiele zu dem erfindungsgemäßen Gläsern Nr. 1 bis 36.

Zur Glasherstellung wurden entweder Grün- oder Sinterkörper in das Schmelzgefäß eingelegt. Als Schmelzgefäß wurde ein hauptsächlich aus Iridium bestehender Tiegel verwendet, der mittels induktiv zugeführter elektrischer Energie beheizt wurde. Der hautsächlich aus Iridium bestehende Tiegel gibt die aufgenommene elektrische Energie mittels Strahlungswärme und direkter Wärmeleitung an das eingelegte Gemenge und/oder die Schmelze ab. Als maximale Schmelztemperatur wurden etwa 2300°C erreicht.

Zur Weiterverarbeitung wurde das flüssige Glas der Schmelze entnommen und in Form von Glasposten erstarren lassen. Die erkalteten Glasposten wurden mit Hilfe des aus der DE 41 00 604 C1 bekannten Verfahrens zu einem Glaspulver mit einer mittleren Korngröße von höchstens 10 µm zermahlen. Die Glaseigenschaften wurden anhand von Glasposten bestimmt, die nicht zu Pulvern zermahlen wurden. Sämtliche Gläser Nr. 1 bis 36 weisen eine hervorragende chemische Beständigkeit gegenüber Säuren, Laugen und Wasser auf; sie sind ferner weitestgehend chemisch inert. Die Brechnungsindices nd und die Dichte D der Varianten des erfindungsgemäßen Glases sind ebenfalls in Tabelle 1 aufgeführt.

Die Vergleichsbeispiele Nr. 37 und 38 enthalten nur Si0₂ und Zr0₂, weisen einen Brechungsindex n_{d} von 1,498 bzw. 1,513 und eine Dichte D von 2,297 g/cm³ bzw. 2,357 g/cm³ auf.

Im Vergleich dazu wurden bei dem Beispiel Nr. 9 2 Mol-% des Zr0₂-Gehalts von Beispiel Nr. 37 durch Yb₂0₃ substituiert. Diese Substitution bewirkt nur eine sehr geringe Änderung des Brechungsindex, aber um eine etwa 6% größere Dichte. Da die Dichte mit der Röntgenopazität korreliert, bedeutet eine große Dichte gleichzeitig eine hohe Röntgenopazität, die für die Anwendung von Gläsern als Füllstoff in Dentalmassen Voraussetzung ist. Das Glas des Beispiels Nr. 9 verbessert folglich die Röntgenopazität gegenüber dem Stand der Technik, ohne die optischen Eigenschaften nennenswert zu verändern.

In Beispiel Nr. 8 wurden im Vergleich zu Beispiel Nr. 37 der Gehalt von SiO₂ um 1,8 Mol-% und der Gehalt von ZrO₂ um 3,2 Mol-% reduziert, während Yb₂O₃ mit einem Gehalt von 5 Mol-% in diesem Glas enthalten war. Als Folge ist ein Zuwachs des Brechungsindex um nur rund 1,7% zu beobachten, während die Dichte um rund 17% verglichen mit Beispiel Nr. 37 zunimmt. Somit ist auch das Glas des Beispiels Nr. 8 hervorragend für den erfindungsgemäßen Zweck einzusetzen.

Das Verhältnis des prozentualen Zuwachses der Dichte eines Beispielglases zu dem prozentualen Zuwachs des Brechungsindex eines Beispielglases jeweils in Bezug zu dem Glas des Beispiels Nr. 37 wird im weiteren "Opazitätsfaktor" Fo genannt. Je größer Fₒ eines erfindungsgemäßen Glases ist, desto günstiger ist im allgemeinen das Verhältnis zwischen vorliegender Brechungsindexänderung und erreichter Dichteerhöhung.

Die Beispiele Nr. 23 bis 28 belegen, daß eine Reduzierung des SiO₂-Gehalts auf 92 Mol-% und des ZrO₂-Gehalts auf 3 Mol-% bei einem Gehalt von zusätzlichen Oxiden von 4,9 Mol-% bei dem Vorhandensein von Yb₂O₃ ebenfalls zu einer Erhöhung der Dichte führen. Allerdings steigt im Fall von Nb₂O₅ und Ta₂O₅ ebenfalls der Brechungsindex stark an. Beispiel Nr. 9 zeigt mit einem Opazitätsfaktor von 17,0 einen sehr guten Kompromiß aus Brechungsindex- und Dichteerhöhung. Darüber hinaus belegt Beispiel Nr. 9 durch den Vergleich mit den Beispielen Nr. 23 bis 28, die einen erheblich geringeren Gehalt an Yb₂O₃ als Beispiel Nr. 9 und Fₒ-Werte zwischen 2,2 und 13,0 aufweisen, daß insbesondere Yb₂O₃ als Bestandteil eines Glases eine effiziente Erhöhung der Dichte und damit der Röntgenopazität ohne eine allzu große Erhöhung des Brechungsindex zur Folge hat.

Um die Auswirkungen des Vorhandenseins von Yb₂O₃ in Glaszusammensetzungen weiter verdeutlichen zu können, wurden die Gläser entsprechend den Beispielen Nr. 1 und 2 erschmolzen. Diese bestehen nur aus SiO₂ und einem relativ großen Anteil an Yb₂O₃. Der Anteil von 18 Mol-% Yb₂O₃ in Beispiel Nr. 1 bewirkt im Vergleich zu Beispiel Nr. 37 einen Anstieg des Brechungsindex um 8,6%, während die Dichte um 64,4% zunimmt. Ein Glas mit einem Gehalt von 25 Mol-% Yb₂O₃ gemäß Beispiel Nr. 2 weist bereits einen um 12,7% größeren Brechungsindex und eine um 89,7% größere Dichte als das Glas des Beispiels Nr. 37 auf. Daß Yb₂O₃ besser als ZrO₂ geeignet ist, wenn eine möglichst große Dichteerhöhung bei einer möglichst geringen Brechungsindexerhöhung eines Glases angestrebt wird, kann durch den Vergleich der vorgenannten Beispiele mit den Beispielen Nr. 3 und 4 belegt werden. In Beispiel Nr. 3 wurde die Hälfte des Yb₂O₃-Gehalts von Beispiel Nr. 1 durch ZrO₂ substituiert. Das erhaltene Glas Nr. 3 weist zwar nur einen Zuwachs von n_{d} um 7,5% im Vergleich zu Beispiel Nr. 37 auf, was an und für sich günstiger wäre, allerdings aber auch nur einen Zuwachs der Dichte um 41,4% verglichen mit Beispiel Nr. 37. Daraus resultiert der Fₒ-Wert von 5,6 für Beispiel Nr. 3, der signifikant niedriger ist als der Fₒ-Wert von 7,5 des Beispiels Nr. 1. Analog ist in Beispiel Nr. 4 die Hälfte des Yb₂O₃-Gehalts von Beispiel Nr. 2 durch ZrO substituiert. Während Beispiel Nr. 2 einen Fₒ-Wert von 7,1 aufzeigt, wird in Beispiel Nr. 4 nur ein Fₒ-Wert von 5,2 erreicht. Selbstverständlich ist der Wert von Fo alleine nicht alleine ausschlaggebend für die möglichen Anwendungsgebiete der erfindungsgemäßen Gläser, da auch die absolute Größe des jeweiligen Brechungsindex hierfür eine Rolle spielen kann.

Daß durch eine geeignete Kombination auch der anderen Bestandteile der erfindungsgemäßen Gläser ein hoch röntgenopakes und niedrigbrechendes Glas erhalten werden kann, das für verschiedene Anwendungszwecke und Gegebenheiten das geeignetste ist, kann insbesondere durch die Beispiele Nr. 29 bis 34 demonstriert werden. Diese enthalten neben 92 Möl-% SiO₂, 2 Mol-% Yb₂O₃ und 3 Mol-% ZrO₂ jeweils ein weiteres der genannten Oxide. Die Brechungsindices dieser Gläser liegen zwischen 1,515 und 1,553 und ihre Dichte zwischen 2,507 g/cm³ und 2,937 g/cm³. Somit werden Fₒ-Werte zwischen 3,5 und 11,5 erreicht.

Die Beispiele 35 und 36 zeigen ein Glas, das ähnlich den bekannten Röntgenopakern Fluorid enthält. Aus Gründen der Vergleichbarkeit mit den übrigen Beispielen werden die'äquivalenten Synthesewerte für La₂F₆ und Yb₂F₆ angegeben. Beispiel Nr. 35 ist abgeleitet von den Beispielen Nr. 29 bis 34, wobei die zusätzliche oxidische Komponente durch La₂F₆ (äquivalenter Synthesewert) ersetzt wurde. Gegenüber Vergleichsbeispiel Nr. 37 ist der Brechungsindex bei diesem Beispiel um rund 1,4% erhöht, während die Dichte um rund 13,4% zunimmt. Fo in Beispiel Nr. 35 beträgt somit etwa 9,8.

In Beispiel Nr. 36 wurde der Yb₂O₃-Gehalt des Beispiels Nr. 9 durch Yb₂F₆ (äquivalenter Synthesewert) ersetzt. Der Brechungsindex dieses Glases ist nur um 0,2% größer als der des Vergleichsbeispiels Nr. 37, die Dichte aber um 5,3%. Daraus berechnet sich ein Wert des Fₒ-Faktors von rund 22,8. Die Verwendung von Ytterbiumfluorid in dem erfindungsgemäßen Glas ist somit besonders vorteilhaft, wenn bei der Verwendung als Füllstoff in Kompositen für die Zahnrestauration hohe Anforderungen an die optischen Eigenschaften des Glases gestellt werden.

Sämtliche Beispiele zeigen auch, daß neben der Einstellung von Brechungsindex und Dichte durch die Abstimmung der Bestandteile in Hinblick auf ihre Röntgenabsorptionsbanden wie zuvor beschrieben eine Anpassung des Glases an die Röntgenquelle möglich ist.

Gegenüber dem Stand der Technik ist das erfindungsgemäße Glas u.a. wegen dem Verzicht auf B₂O₃ und/oder Al₂O₃ weitestgehend chemisch inert. Es weist eine verbesserte Röntgenopazität auf und seine Brechungsindices können in einem angemessenen Bereich um 1,498 an den Anwendungszweck angepaßt werden. Dadurch ist es in vorteilhafter Weise insbesondere als Füllstoffe in Dentalmassen, aber auch für andere Anwendungen, welche hohe Anforderungen u.a. an die Reinheit sowie die chemische und die Temperaturbeständigkeit stellen, verwendet werden. Durch das erfindungsgemäße Verfahren kann es kostengünstig großtechnisch hergestellt werden.

**Tabelle 1**

| Zusammensetzungen des röntgenopaken Glases in Mol-% | | | | | | | | | | | | | | | | | | | |
|---|---|---|---|---|---|---|---|---|---|---|---|---|---|---|---|---|---|---|---|
| **Beispiel Nr*.*** | **1** | **2** | **3** | **4** | **5** | **6** | **7** | **8** | **9** | **10** | **11** | **12** | **13** | **14** | **15** | **16** | **17** | **18** | **19** |
| SiO₂ | 82 | 75 | 82 | 75 | 75 | 88,1 | 88,1 | 92 | 93,8 | 88,1 | 88,1 | 88,1 | 88,1 | 88,1 | 88,1 | 82 | 88,1 | 88,1 | 88,1 |
| Yb₂O₃ | 18 | 25 | 9 | 12,5 | 12,5 | 5 | 10 | 5 | 2 | 0,1 | 0,1 | 0,1 | 0,1 | 0,1 | 0,1 | 6 | 0,1 | 0,1 | 0,1 |
| ZrO₂ | | | 9 | 12,5 | 6,25 | 6,9 | 1,9 | 3 | 4,2 | 6,9 | 6,9 | 6,9 | 6,9 | 6,9 | 6,9 | 6 | 1,9 | 1,9 | 1,9 |
| Nb₂O₅ | | | | | | | | | | 4,9 | | | | | | | 9,9 | | |
| Ta₂O₅ | | | | | | | | | | | 4,9 | | | | | | | 9,9 | |
| HfO₂ | | | | | | | | | | | | 4,9 | | | | | | | 9,9 |
| WO₃ | | | | | | | | | | | | | 4,9 | | | | | | |
| La₂O₃ | | | | | 6,25 | | | | | | | | | 4,9 | | | | | |
| Y₂O₃ | | | | | | | | | | | | | | | 4,9 | 6 | | | |
| La₂F₆ | | | | | | | | | | | | | | | | | | | |
| Yb₂F₆ | | | | | | | | | | | | | | | | | | | |
| n_{d} | 1,626 | 1,687 | 1,609 | 1,668 | 1,672 | 1,581 | 1,591 | 1,524 | 1,503 | 1,631 | 1,621 | 1,571 | 1,581 | 1,571 | 1,571 | 1,602 | 1,681 | 1,661 | 1,561 |
| D | 3,777 | 4,357 | 3,247 | 3,667 | 3,797 | 3,044 | 3,364 | 2,687 | 2,428 | 2,894 | 3,444 | 2,914 | 2,894 | 2,854 | 2,744 | 3,077 | 3,054 | 4,084 | 3,104 |
| Fₒ | 7,5 | 7,1 | 5,6 | 5,2 | 5,6 | 5,8 | 7,4 | 9,6 | 17,0 | 2,9 | 6,1 | 5,5 | 4,7 | 4,9 | 4,0 | 4,9 | 2,7 | 7,1 | 8,3 |
| SiO₂ | 88,1 | 88,1 | 88,1 | 92 | 92 | 92 | 92 | 92 | 92 | 92 | 92 | 92 | 92 | 92 | 92 | 92 | 93,8 | 93,8 | 92 |
| Yb₂O₃ | 0,1 | 0,1 | 0,1 | 0,1 | 0,1 | 0,1 | 0,1 | 0,1 | 0,1 | 2 | 2 | 2 | 2 | 2 | 2 | 2 | | | |
| ZrO₂ | 1,9 | 1,9 | 1,9 | 3 | 3 | 3 | 3 | 3 | 3 | 3 | 3 | 3 | 3 | 3 | 3 | 3 | 4,2 | 6,2 | 8 |
| Nb₂O₅ | | | | 4,9 | | | | | | 3 | | | | | | | | | |
| Ta₂O₅ | | | | | 4,9 | | | | | | 3 | | | | | | | | |
| HfO₂ | | | | | | 4,9 | | | | | | 3 | | | | | | | |
| WO₃ | 9,9 | | | | | | 4,9 | | | | | | 3 | | | | | | |
| La₂O₃ | | 9,9 | | | | | | 4,9 | | | | | | 3 | | | | | |
| Y₂O₃ | | | 9,9 | | | | | | 4,9 | | | | | | 3 | | | | |
| La₂F₆ | | | | | | | | | | | | | | | | 3 | | | |
| Yb₂F₆ | | | | | | | | | | | | | | | | | 2 | | |
| n_{d} | 1,581 | 1,581 | 1,571 | 1,573 | 1,564 | 1,51 | 1,522 | 1,519 | 1,514 | 1,553 | 1,549 | 1,515 | 1,523 | 1,521 | 1,518 | 1,518 | 1,501 | 1,498 | 1,513 |
| D | 3,074 | 2,984 | 2,764 | 2,547 | 3,097 | 2,547 | 2,537 | 2,487 | 2,377 | 2,597 | 2,937 | 2,607 | 2,597 | 2,567 | 2,507 | 2,605 | 2,419 | 2,297 | 2,357 |
| Fₒ | 6,1 | 5,4 | 4,1 | 2,2 | 7,8 | 13,0 | 6,4 | 5,8 | 3,2 | 3,5 | 8,1 | 11,5 | 7,7 | 7,5 | 6,7 | 9,8 | 22,7 | | |

## Patentansprüche

1. Röntgenopakes Al₂O₃- und B₂O₃-freies Dentalglas, **gekennzeichnet durch** eine Zusammensetzung in Mol-% von:
| | |
|---|---|
| SiO₂ | 60 - 98 |
| Yb₂O₃ | 0,1 - 40 |
| ZrO₂ | 0 - 40 |
| WO₃ | 0 - 40 |
| La₂O₃ | 0 - 40 |
| Nb₂O₅ | 0 - 40 |
| HfO₂ | 0 - 40 |
| Ta₂O₅ | 0 - 40 |
| Gd₂O₃ | 0 - 40 |
| Lu₂O₃ | 0 - 40 |
| Sc₂O₃ | 0 - 40 |
| Y₂O₃ | 0 - 40 |
| F₂ | 0 - 5 |
| Li₂O | 0 - <10 |
| Na₂O | 0 - <10 |
| K₂O | 0 - <10 |
| MgO | 0 - 10 |
| CaO | 0 - 10 |
| SrO | 0 - 10 |
| BaO | 0 - 10 |
| ZnO | 0 - 10 |
| TiO₂ | 0 - 10 |
| GeO₂ | 0 - 10 |
| P₂O₅ | 0 - 10 |
| mit | |
| ∑ Li₂O + Na₂O + K₂O | 0 - <10 |
| ∑ MgO + CaO + SrO + BaO | 0 - <10 |
| ∑ TiO₂ + GeO₂ + P₂O₅ | 0 - <15 |

2. Röntgenopakes Al₂O₃- und B₂O₃-freies Dentalglas nach Anspruch 1 mit einem Gehalt in Mol-% von:
| | |
|---|---|
| SiO₂ | 60 - 98 |
| Yb₂O₃ | 0,1 - 40 |
| ZrO₂ | 0,1 - 40 |

3. Röntgenopakes Al₂O₃- und B₂O₃-freies Dentalglas nach Anspruch 1 mit einem Gehalt in Mol-% von:
| | |
|---|---|
| SiO₂ | 70 - 98 |
| Yb₂O₃ | 0,5 - 15 |
| ZrO₂ | 0,5 - 15 |

4. Röntgenopakes Al₂O₃- und B₂O₃-freies Dentalglas nach Anspruch 1 mit einem Gehalt in Mol-% von:
| | |
|---|---|
| SiO₂ | 70 - 98 |
| Yb₂O₃ | 1 - 15 |
| ZrO₂ | 1 - 15 |

5. Röntgenopakes Al₂O₃- und B₂O₃-freies Dentalglas nach mindestens einem der Ansprüche 1 bis 4, **gekennzeichnet durch** eine Zusammensetzung, die höchstens fünf oxidische Komponenten beinhaltet.

6. Röntgenopakes Al₂O₃- und B₂O₃-freies Dentalglas nach mindestens einem der Ansprüche 1 bis 4, **gekennzeichnet durch** eine Zusammensetzung, die höchstens vier oxidische Komponenten beinhaltet.

7. Röntgenopakes Al₂O₃- und B₂O₃-freies Dentalglas nach mindestens einem der Ansprüche 1 bis 4, **gekennzeichnet durch** eine Zusammensetzung, die höchstens drei oxidische Komponenten beinhaltet.

8. Al₂O₃- und B₂O₃-freies Dentalglaspulver mit einer mittleren Korngröße bis zu 20 µm, **gekennzeichnet durch** eine Zusammensetzung nach mindestens einem der Ansprüche 1 bis 7.

9. Al₂O₃- und B₂O₃-freies Dentalglaspulver nach Anspruch 8, **gekennzeichnet durch** eine Silanisierung seiner Oberfläche.

10. Verfahren zur Herstellung eines Al₂O₃- und B₂O₃-freien Dentalglases mit einer Zusammensetzung nach mindestens einem der Ansprüche 1 bis 7, umfassend die Gemengeaufbereitung aus den Rohstoffkomponenten des Glases, die Gemengeeinlage und das Schmelzen in einem Schmelzgefäß, **dadurch gekennzeichnet, daß** die Temperatur beim Schmelzen mindestens 1500°C, besonders bevorzugt mindestens 1600°C beträgt.

11. Verfahren nach Anspruch 10, **dadurch gekennzeichnet, daß** das Schmelzgefäß zumindest teilweise aus massivem Iridium und/oder Legierungen mit hohem Iridiumgehalt besteht.

12. Verfahren nach Anspruch 10, **dadurch gekennzeichnet, daß** das Schmelzen mit Hilfe der Einstrahlung von Hochfrequenz erfolgt.

13. Verfahren nach Anspruch 12, **dadurch gekennzeichnet, daß** die Hochfrequenz von 50kHz bis 2 MHz beträgt.

14. Verfahren nach mindestens einem der Ansprüche 10 bis 13, **dadurch gekennzeichnet, daß** zumindest eine Rohstoffkomponente des Glases vor der Gemengeeinlage als nanoskaliges Pulver vorliegt.

15. Verfahren nach mindestens einem der Ansprüche 10 bis 14, **dadurch gekennzeichnet, daß** für die Gemengeaufbereitung zumindest eine Rohstoffkomponente als nanoskaliges Pulver vorliegt, das zusammen mit den übrigen Rohstoffkomponenten in einem Lösungsmittel dispergiert und/oder gelöst, in eine Form eingebracht und zu einem Grünkörper getrocknet wird.

16. Verfahren nach Anspruch 15, **dadurch gekennzeichnet, daß** die Trocknung der in die Form eingebrachten dispergierten und/oder gelösten Rohstoffkomponenten mit Hilfe der Einwirkung von Mikrowellenstrahlung erfolgt.

17. Verfahren nach Anspruch 16, **dadurch gekennzeichnet, daß** die Form zumindest teilweise aus einem nicht benetzenden Material, bevorzugt Teflon, besteht.

18. Verfahren nach mindestens einem der Ansprüche 15 bis 17, **dadurch gekennzeichnet, daß** der Grünkörper als Ganzes oder gemahlen als Gemenge eingelegt wird.

19. Verfahren nach mindestens einem der Ansprüche 15 bis 17, **dadurch gekennzeichnet, daß** der Grünkörper gemahlen, in einem Lösungsmittel dispergiert und/oder gelöst und zu einem Kompaktkörper getrocknet wird.

20. Verfahren nach Anspruch 15 und/oder 19, **dadurch gekennzeichnet, daß** als Lösungsmittel Alkalilauge oder Ammoniakwasser verwendet wird.

21. Verfahren nach mindestens einem der Ansprüche 15 bis 20, **dadurch gekennzeichnet, daß** der Grünkörper und/oder der Kompaktkörper gesintert werden.

22. Verfahren nach Anspruch 21, **dadurch gekennzeichnet, daß** die Abwärme des Schmelzens zumindest teilweise für die Sinterung verwendet wird.

23. Verwendung eines Glases nach mindestens einem der Ansprüche 1 bis 7 als Füllstoff in Kompositen für die Zahnrestauration.

24. Verwendung nach Anspruch 23 als Füllstoff in Kompositen auf Epoxydharzbasis.

25. Verwendung eines Glases nach mindestens einem der Ansprüche 1 bis 7 als Röntgenopaker in Dentalmassen.

## Claims

1. X-ray opaque Al₂O₃- and B₂O₃-free dental glass, **characterized by** a composition in mol% of:
| | |
|---|---|
| SiO₂ | 60-98 |
| Yb₂O₃ | 0.1-40 |
| ZrO₂ | 0-40 |
| WO₃ | 0-40 |
| La₂O₃ | 0-40 |
| Nb₂O₅ | 0-40 |
| HfO₂ | 0-40 |
| Ta₂O₅ | 0-40 |
| Gd₂O₃ | 0-40 |
| Lu₂O₃ | 0-40 |
| Sc₂O₃ | 0-40 |
| Y₂O₃ | 0-40 |
| F₂ | 0-5 |
| Li₂O | 0-<10 |
| Na₂O | 0-<10 |
| K₂O | 0-<10 |
| MgO | 0 - 10 |
| CaO | 0 - 10 |
| SrO | 0 - 10 |
| BaO | 0 - 10 |
| ZnO | 0 - 10 |
| TiO₂ | 0 - 10 |
| GeO₂ | 0 - 10 |
| P₂O₅ | 0 - 10 |
with
| | |
|---|---|
| ∑ MgO + CaO + SrO + BaO | 0 -<10 |
| ∑ Li₂O + Na₂O + K₂O | 0-<10 |
| ∑ TiO₂ + GeO₂ + P₂O₅ | 0 -<15 |

2. X-ray opaque Al₂O₃- and B₂O₃-free dental glass according to Claim 1, having a content in mol% of:
| | |
|---|---|
| SiO₂ | 60-98 |
| Yb₂O₃ | 0.1-40 |
| ZrO₂ | 0.1-40 |

3. X-ray opaque Al₂O₃- and B₂O₃-free dental glass according to Claim 1, having a content in mol% of:
| | |
|---|---|
| SiO₂ | 70-98 |
| Yb₂O₃ | 0.5-15 |
| ZrO₂ | 0.5-15 |

4. X-ray opaque Al₁O₃- and B₂O₃-free dental glass as claimed in Claim 1, having a content in mol% of:
| | |
|---|---|
| SiO₂ | 70-98 |
| Yb₂O₃ | 1-15 |
| ZrO₂ | 1-15 |

5. X-ray opaque Al₂O₃- and B₂O₃-free dental glass as claimed in at least one of Claims 1 to 4, **characterized by** a composition which contains at most five oxidic components.

6. X-ray opaque Al₂O₃- and B₂O₃-free dental glass as claimed in at least one of Claims 1 to 4, **characterized by** a composition which contains at most four oxidic components.

7. X-ray opaque Al₂O₃- and B₂O₃-free dental glass as claimed in at least one of Claims 1 to 4, **characterized by** a composition which contains at most three oxidic components.

8. Al₂O₃- and B₂O₃-free dental glass powder with a mean grain size of up to 20 µm, **characterized by** a composition as claimed in at least one of Claims 1 to 7.

9. Al₂O₃- and B₂O₃-free dental glass powder as claimed in Claim 8, **characterized by** silanization of its surface.

10. Process for producing an Al₂O₃- and B₂O₃-free dental glass having a composition as claimed in at least one of Claims 1 to 7, comprising batch preparation from the raw material components of the glass, batch charge and melting in a melting vessel, **characterized in that** the temperature during melting is at least 1500°C, particularly preferably at least 1600°C.

11. Process as claimed in Claim 10, **characterized in that** the melting vessel at least partially comprises solid iridium and/or alloys with a high iridium content.

12. Process as claimed in Claim 10, **characterized in that** the melting is carried out with the aid of incident high-frequency radiation.

13. Process as claimed in Claim 12, **characterized in that** the high frequency is from 50 kHz to 2 MHz.

14. Process as claimed in at least one of Claims 10 to 13, **characterized in that** at least one raw material component of the glass is in the form of nanoscale powder prior to the step of batch charge.

15. Process as claimed in at least one of Claims 10 to 14, **characterized in that** for the batch preparation at least one raw material component is in the form of nanoscale powder which is dispersed and/or dissolved in a solvent together with the remaining raw material components, introduced into a mold and dried to form a green body.

16. Process as claimed in Claim 15, **characterized in that** the drying of the raw material components which have been dissolved and/or dispersed and introduced into the mold is carried out with the aid of the action of microwave radiation.

17. Process as claimed in Claim 16, **characterized in that** the mold at least partially comprises a non-wetting material, preferably Teflon.

18. Process as claimed in at least one of Claims 15 to 17, **characterized in that** the green body is charged as batch either as a single entity or in milled form.

19. Process as claimed in at least one of Claims 15 to 17, **characterized in that** the green body is milled, dissolved and/or dispersed in a solvent and dried to form a compact body.

20. Process as claimed in Claim 15 and/or 19, **characterized in that** the solvent used is alkali metal lye or ammonia water.

21. Process as claimed in at least one of Claims 15 to 20, **characterized in that** the green body and/or the compact body are sintered.

22. Process as claimed in Claim 21, **characterized in that** the waste heat of melting is at least partially used for the sintering.

23. Use of a glass as claimed in at least one of Claims 1 to 7 as a filler in composites for dental restoration.

24. Use according to Claim 23 as a filler in composites based on epoxy resin.

25. Use of a glass as claimed in at least one of Claims 1 to 7 as an X-ray opacifier in dental compositions.

## Revendications

1. Verre dentaire radio-opaque exempt d'Al₂O₃ et de B₂O₃, **caractérisé par** une composition, en % en moles de :
| | |
|---|---|
| SiO₂ | 60 - 98 |
| Yb₂O₃ | 0,1 - 40 |
| ZrO₂ | 0 - 40 |
| WO₃ | 0 - 40 |
| La₂O₃ | 0 - 40 |
| Nb₂O₅ | 0 - 40 |
| HfO₂ | 0 - 40 |
| Ta₂O₅ | 0 - 40 |
| Gd₂O₃ | 0 - 40 |
| Lu₂O₃ | 0 - 40 |
| Sc₂O₃ | 0 - 40 |
| Y₂O₃ | 0 - 40 |
| F₂ | 0 - 5 |
| Li₂O | 0 - <10 |
| Na₂O | 0 - <10 |
| K₂O | 0 - <10 |
| MgO | 0 - 10 |
| CaO | 0 - 10 |
| SrO | 0 - 10 |
| BaO | 0 - 10 |
| ZnO | 0 - 10 |
| TiO₂ | 0 - 10 |
| GeO₂ | 0 - 10 |
| P₂O₅ | 0 - 10 |
avec
| | |
|---|---|
| ∑ Li₂O + Na₂O + K₂O | 0 - <10 |
| ∑ MgO + CaO + SrO + BaO | 0 - <10 |
| ∑ TiO₂ + GeO₂ + P₂O₅ | 0 - <15 |

2. Verre dentaire radio-opaque exempt d'Al₂O₃ et de B₂O₃ selon la revendication 1, ayant une teneur, en % en moles, de :
| | |
|---|---|
| SiO₂ | 60 - 98 |
| Yb₂O₃ | 0,1 - 40 |
| ZrO₂ | 0,1 - 40 |

3. Verre dentaire radio-opaque exempt d'Al₂O₃ et de B₂O₃ selon la revendication 1, ayant une teneur, en % en moles de :
| | |
|---|---|
| SiO₂ | 70 - 98 |
| Yb₂O₃ | 0,5 - 15 |
| ZrO₂ | 0,5 - 15 |

4. Verre dentaire radio-opaque exempt d'Al₂O₃ et de B₂O₃ selon la revendication 1, ayant une teneur, en % en moles, de :
| | |
|---|---|
| SiO₂ | 70 - 98 |
| Yb₂O₃ | 1 - 15 |
| ZrO₂ | 1 - 15 |

5. Verre dentaire radio-opaque exempt d'Al₂O₃ et de B₂O₃ selon au moins l'une des revendications 1 à 4, **caractérisé par** une composition qui contient au plus cinq composants de type oxyde.

6. Verre dentaire radio-opaque exempt d'Al₂O₃ et de B₂O₃ selon au moins l'une des revendications 1 à 4, **caractérisé par** une composition qui contient au plus quatre composants de type oxyde.

7. Verre dentaire radio-opaque exempt d'Al₂O₃ et de B₂O₃ selon au moins l'une des revendications 1 à 4, **caractérisé par** une composition qui contient au plus trois composants de type oxyde.

8. Poudre de verre dentaire exempte d'Al₂O₃ et de B₂O₃, ayant une granulométrie moyenne allant jusqu'à 20 µm, **caractérisée par** une composition selon au moins l'une des revendications 1 à 7.

9. Poudre de verre dentaire exempte d'Al₂O₃ et de B₂O₃ selon la revendication 8, **caractérisée par** une silanisation de sa surface.

10. Procédé de fabrication d'un verre dentaire exempt d'Al₂O₃ et de B₂O₃, ayant une composition selon au moins l'une des revendications 1 à 7, comprenant la préparation d'un mélange vitrifiable à partir des composants de la matière première du verre, l'enfournement du mélange vitrifiable et la fusion dans un récipient de fusion, **caractérisé en ce que** la température, lors de la fusion, est d'au moins 1500°C, d'une manière particulièrement préférée d'au moins 1600°C.

11. Procédé selon la revendication 10, **caractérisé en ce que** le récipient de fusion est au moins partiellement constitué d'iridium massif et/ou d'alliages ayant une teneur élevée en iridium.

12. Procédé selon la revendication 10, **caractérisé en ce que** la fusion a lieu par une irradiation à haute fréquence.

13. Procédé selon la revendication 12, **caractérisé en ce que** la haute fréquence est de 50 kHz à 2 MHz.

14. Procédé selon au moins l'une des revendications 10 à 13, **caractérisé en ce qu'**au moins un composant de la matière première du verre se présente, avant enfournement du mélange vitrifiable, sous forme d'une poudre nanoscalaire.

15. Procédé selon au moins l'une des revendications 10 à 14, **caractérisé en ce que**, pour la préparation du mélange vitrifiable, au moins un composant de la matière première se présente sous forme d'une poudre nanoscalaire, qui en même temps que les autres composants de la matière première est dispersée et/ou dissoute dans un solvant, placée dans un moule, et séchée pour donner une ébauche crue.

16. Procédé selon la revendication 15, **caractérisé en ce que** le séchage des composants de la matière première dispersés et/ou dissous, introduits dans le moule, s'effectue sous l'action d'un rayonnement micro-ondes.

17. Procédé selon la revendication 16, **caractérisé en ce que** le moule est constitué au moins en partie d'un matériau non mouillable, de préférence le Téflon.

18. Procédé selon au moins l'une des revendications 15 à 17, **caractérisé en ce que** l'ébauche crue est enfournée en bloc, ou broyée sous forme d'un mélange vitrifiable.

19. Procédé selon au moins l'une des revendications 15 à 17, **caractérisé en ce que** l'ébauche crue est broyée, dispersée et/ou dissoute dans un solvant, et séchée pour donner un comprimé.

20. Procédé selon la revendication 15 et/ou 19, **caractérisé en ce qu'**on utilise en tant que solvant une lessive alcaline ou de l'ammoniaque.

21. Procédé selon au moins l'une des revendications 15 à 20, **caractérisé en ce que** l'ébauche crue et/ou le comprimé sont frittés.

22. Procédé selon la revendication 21, **caractérisé en ce que** la chaleur perdue de la fusion est au moins en partie utilisée pour le frittage.

23. Utilisation d'un verre selon au moins l'une des revendications 1 à 7 en tant que produit d'obturation dans des composites pour la restauration dentaire.

24. Utilisation selon la revendication 23 en tant que produit d'obturation dans des composites à base d'une résine époxyde.

25. Utilisation d'un verre selon au moins l'une des revendications 1 à 7 en tant que produit radio-opaque dans des masses dentaire.
